Europäisches Patentamt

European Patent Office

Office européen des brevets

(11) Publication number: **0 461 060 A1**

## (12) EUROPEAN PATENT APPLICATION

(21) Application number: 91500052.5

(22) Date of filing: 04.06.91

(51) Int. Cl.5: **B65D 47/28**, A61M 5/32, A61B 19/02

(30) Priority: 05.06.90 ES 9001760

(43) Date of publication of application:
11.12.91 Bulletin 91/50

(84) Designated Contracting States:
AT BE CH DE DK FR GB GR IT LI LU NL SE

(71) Applicant: Argüeso Gama, Francisco Javier
Pol. Ind. Elbarrera S/N
E-20150 Aduna, Guipuzcoa(ES)

(72) Inventor: Argüeso Gama, Francisco Javier
Pol. Ind. Elbarrera S/N
E-20150 Aduna, Guipuzcoa(ES)

(74) Representative: Ungria Lopez, Javier et al
Avda. Ramon y Cajal, 78
E-28043 Madrid(ES)

(54) **Box for removing and containing hypodermic needles.**

(57) The box has the shape of a conventional closed container (1) with a hole (3) in its top part, over which it is possible to place opposite it another hole (4) belonging to a cover (2) mounted on the top in a sliding manner with regard to the body (1) of the container or box. The cover (2) is placed towards a certain position, by means of a spring (8) in which the holes (3) and (4) remain out of step between each other in order to prevent the coming out of any hypodermic needle that has been previously inserted through the confronting of the two holes. Said confronting is achieved by pushing the cover by hand and said cover slides in a guided manner.

By means of the cited box it is possible to separate hypodermic needles from the syringes without humans touching the former preventing contagion and infections of sanitary personnel.

FIG.I

## OBJECT OF THE INVENTION

As is expressed in the title of this specification, the present invention refers to a box for removing and containing hypodermic needles, to be used by sanitary personnel in hospitals and clinics as well as for individual use by said personnel (doctors and nurses), who go to patients' homes.

The purpose of the box is to carry out the removal of hypodermic needles from the corresponding syringes after medical use thereof, and to remain automatically inside the airtight vessel, without in said processes there being any human contact with the hypodermic needles, thus avoiding contagion and infection for the users thereof.

## BACKGROUND OF THE INVENTION

Presently some containers for the removal of hypodermic needles from syringes are known. These containers will obviously contain the needles that have been made independent from their syringes.

The type of container that we are referring to tends to be cylindric or prismatic bodies with a mouth determined by radial cuts or the like that determine flexible fins that permit the needle to be introduced and that upon pulling the syringe the former is retained falling inside the vessel.

Undoubtedly, this type of container is provided for general use thereof, due to the size of the same and they are not a container which can be used personally, thus, the sanitary personnel when he goes to patients' homes cannot take it with him as the container is big and thus it cannot even be carried permanently around the different patients' rooms in a hospital or clinic.

## DESCRIPTION OF THE INVENTION

The box of the invention is provided for use permanently by medical personnel in an individual manner, in other words, each doctor or nurse can carry the box permanently with himself and when a hypodermic needle is used after the corresponding injection given to a patient the needle can be removed by the box itself and remain contained in the same, without the least amount of contact by the sanitary personnel.

From what has been said up until now it is inferred that the box will be relatively small, easy to handle for any size hand. It can even be transported in the user's pocket without the same running the risk of contact with the content, given that the box is airtight and given the resistance of its walls to be penetration of the needles it contains.

When the user considers that the vessel or box is full of hypodermic needles, he should subject the same to total destruction thereof by incineration, thus the container is totally destroyed and volatized and the contents are destroyed and disinfected.

Structurally, the box is formed by two bodies, one acting as the container or box itself and the other acting as a cover, the latter being moveable with regard to the former and put towards a certain position by the action of a spring.

The body of the box or container has a general prismatic rectangular shape with rounded edges and in whose top part there is a deep shoulder with reduction of the height in a long section of that top part, in such a way that between the wall corresponding to the shoulder and the end wall or wall facing the cover, there is the spring that maintains said cover towards a so-called closing position, and in whose position a hole of said cover remains out of step with regard to the other hole foreseen for that purpose in the body of the box, while by hand pressure against the action of the spring and by means of the corresponding movement of the cover one manages to put the holes opposite each other in order to introduce the hypodermic needle that can be easily removed simply by releasing the cover which will return to its original position by means of the spring, logically pulling the syringe that holds the hypodermic needle.

Logically, sideways the body of the box has some guides for sliding of the corresponding cover, guides formed by some side channels or grooves in which there are projections belonging to the bottom edge of the sides of the cover itself.

In order to complete the description that is going to be made hereinafter and for the purpose of providing a better understanding of the features of the invention, the present specification is accompanied by a set of drawings on the basis of whose figures one will more easily understand the innovations and advantages of the box object of the invention.

## BRIEF DESCRIPTION OF THE DRAWINGS

Figure 1.- It shows a raised longitudinal sectioned view of the box object of the invention.

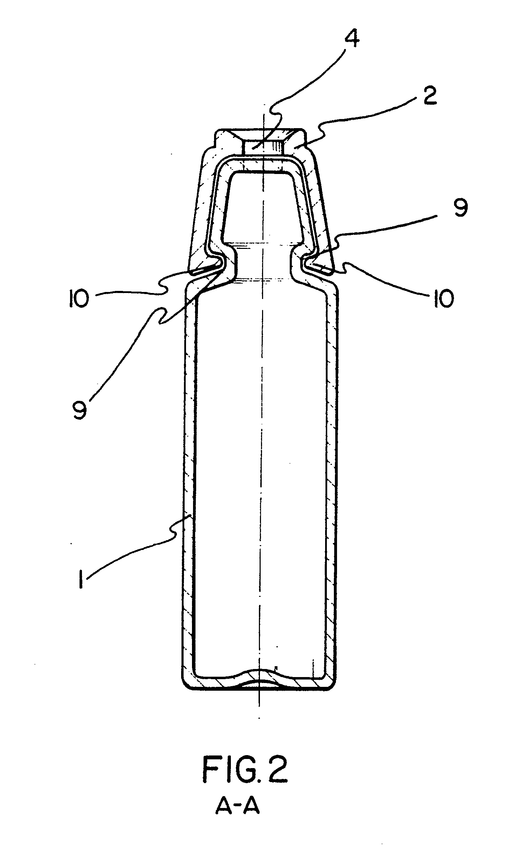

Figure 2.- It shows a raised view of the side of the same box, corresponding to section A-A of figure 1.

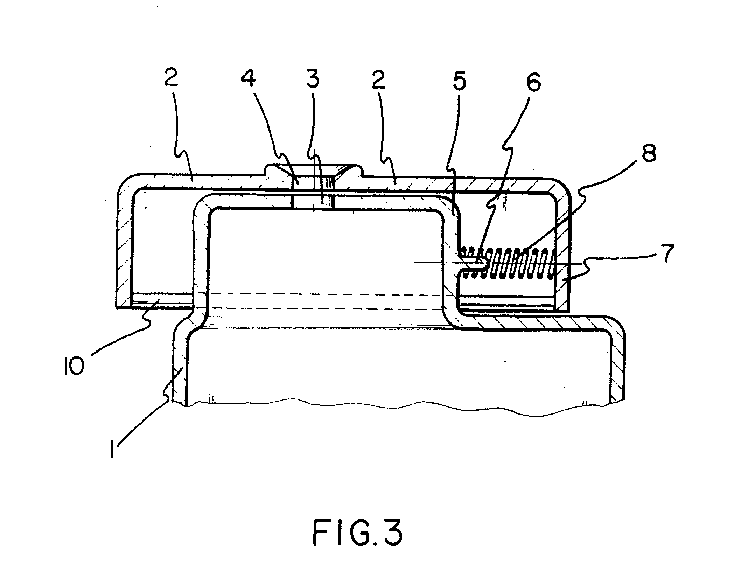

Figure 3.- It shows another section of the top part of the box in which the cover is moved against the action of the spring, the holder of the latter opposite the hole of the box in order to permit the introduction of the hypodermic needles.

## DESCRIPTION OF THE PREFERRED EMBODIMENT

In view of the commented figures, one can observe how the box that the invention proposes on the whole consists of a body (1) forming a container or box itself, and a cover (2) placed on top of the former and capable of moving longitudinally, as will be seen later on.

The body of box (1) has in its base or top part a hole (3) capable of being opposite a hole (4) provided for this purpose in the cover (2.)

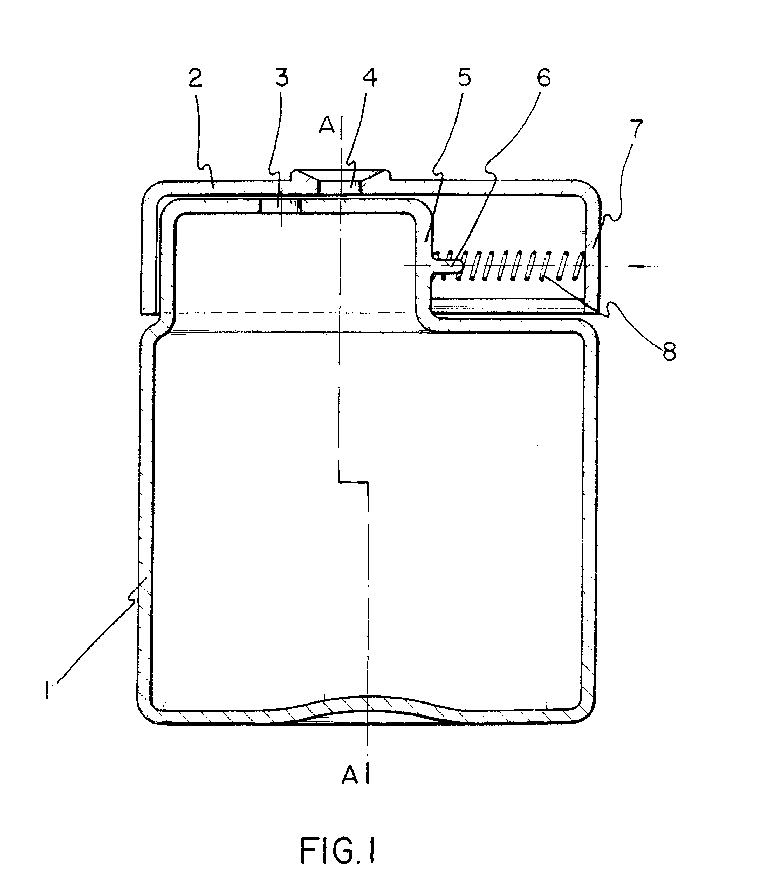

Both bodies (1) and (2) are made out of plastic or resistant material, which is provided so that the needles cannot go through the walls that make up the same, the shape of the body or box (1) preferably being prismatic-rectangular with rounded edges and having on the top a shoulder that gives rise to a lower side part which affects the entire width, whose end wall (5) has an appendix (6) between which and the wall (7) of the cover there is a spring (8) which tends to the cover (2) occupying a position like the one shown in figure 1, in other words, a position in which the holes (3) and (4) remain out of step.

The cover (2) in its movement over the top part of the box (1) is guided on some side grooves or channels (9) in which there are appendixes or projections (10) provided in the side walls themselves of the cover (2) as seen in figure (2.)

The confronting of the holes (3) and (4) cited above makes it possible to insert hypodermic needles inside the box (1) in order to carry out the removal of the same with regard to syringes.

The permanent closing of the hole (3) of the box (1) and therefore the total impossibility of the needles coming out by themselves remains guaranteed by the action of spring (8) which keeps the cover (2) constantly .moved towards the position that is seen in figure 1, in other words, in the position in which hole (3) remains out of step with regard to hole (4) of the cover and therefore closing the former, in such a way that in order to effect the confronting it is necessary to press on the cover (2) against the spring (8) until holes (3) and (4) are opposite each other.

According to the described structure, the operation thereof is as follows:

The user picking up the described unit, in vertical position, as is shown in the cited figures and it being understood that in the other hand the user has a syringe logically provided with a corresponding hypodermic needle which has already been used, he must proceed in the following manner: he must first push against the wall (7) of the cover (2) against the action of the spring (8), until holes (3) and (4) are opposite each other, at which time the hypodermic needle can be inserted through said holes until contact is made with the base of the syringe.

The hypodermic needle being already inserted, through the holes (3) and (4) the user must increase the pressure that had been exerted with the thumb on the moveable cover (2) until the needle remains firmly fastened and pressed between the tangents of both holes (3) and (4), and this permits the hypodermic needle to become independent from the syringe.

Having achieved this separation and the syringe being removed, the needle will still remain pressed between both holes, until the user stops pressing on the moveable cover (2) with his thumb.

Once there is no more pressure on the cover (2), it will begin its reverse movement by means of the action of the spring (8), thus, there will be a total diametric coincidence of both holes (3) and (4) permitting the total freeing of the hypodermic needle which by gravity will be automatically introduced inside the vessel.

Once the needle has been introduced inside the vessel, the pressure will be reduced that has been still exerted by the user on the moveable cover (2) until one finally stops pressing thereon, when said cover (2) will have reached its final position in its reverse movement to the pressure exerted by the user, achieving the inoperative position between the body and the cover, which guarantees the lack of coincidence between holes (3) and (4), thanks to the pressure exerted by spring (8) in a permanent fashion.

In this way all the needles that one desires may be inserted, the vessel or box (1) always remaining closed and holes (3) and (4) are made to coincide when a needle is inserted, in such a way that when said box is full, it will be destroyed by incineration or by any other means that achieves the corresponding volatilization and disinfection.

Finally it should be said that the box can be made out of a base body like the one described, and a cover that is placed inside the base body, instead of being outside as it has been described in the present description.

## Claims

1. Box for removing and containing hypodermic needles, which being useable by sanitary personnel in clinics and hospitals as well as in individual private visits to patients, essentially characterized because it is comprised of two bodies (1) and (2) one acting as a container and the other acting as a cover, the latter being moveable with regard to the body of the container (1) with the particular feature that said cover (2) is mounted on the top part of the body (1) and guided in its movement by projections (10) of its side walls that are positioned and guided in channels or grooves (9) provided for this purpose in the side walls of the body itself (1) of the vessel or body itself, with the particular feature that the top part of the latter has a hole (3) capable of facing the other hole (4) provided for this purpose in the cover (2), both remaining permanently out of step by the action of a spring (8) that presses on and pushes the cover (2) towards the out of step position between holes (3) and (4), it being provided that the confronting of the holes makes it possible to introduce the corresponding hypodermic needle and its becoming independent from the respective syringe.

2. Box for removing and containing hypodermic needles, according to claim 1, characterized because in the top part of the body of box (1) there is a shoulder all along its width and part of its length, determining a vertical wall (5) between which and the wall itself (7) of the box remains spring (8) which pushes on the cover (2.)

FIG.I

**FIG. 2**
A-A

FIG.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WO-A-8 601 700 (POLYTOP)<br>* page 12, line 19 - page 12, line 28; figures 18,19 *<br>– – – | 1,2 | B 65 D 47/28<br>A 61 M 5/32<br>A 61 B 19/02 |
| X | US-A-2 187 735 (ERB)<br>* figures *<br>– – – | 1,2 | |
| A | FR-A-2 603 872 (BONNEVAY)<br>* the whole document *<br>– – – – – | 1,2 | |

|  |  |
|---|---|
| | TECHNICAL FIELDS SEARCHED (Int. Cl.5) |
| | B 65 D<br>A 61 M<br>A 61 B |

**The present search report has been drawn up for all claims**

| Place of search | Date of completion of search | Examiner |
|---|---|---|
| The Hague | 22 August 91 | NEWELL P.G. |